(19) Europäisches Patentamt
European Patent Office
Office européen des brevets

(11) Publication number: 0 325 043 A1

(12) **EUROPEAN PATENT APPLICATION**

(21) Application number: 88311972.9

(22) Date of filing: 16.12.88

(51) Int. Cl.⁴: C07D 239/34 , C07D 239/52 , C09K 19/34

(30) Priority: 23.12.87 JP 325742/87

(43) Date of publication of application: 26.07.89 Bulletin 89/30

(84) Designated Contracting States: DE FR GB IT

(71) Applicant: Chisso Corporation
6-32, Nakanoshima 3-chome Kita-ku
Osaka-shi Osaka-fu(JP)

(72) Inventor: Ushioda, Makoto
12-16, Kannon 1-chome Kawasaki-ku
Kawasaki-shi Kanagawa-ken(JP)
Inventor: Ohno, Kouji
10-3, Otsutomo-cho Kanazawa-ku
Yokohama-shi Kanagawa-ken(JP)
Inventor: Saito, Shinichi
10-1, Otsutomo-cho Kanazawa-ku
Yokohama-shi Kanagawa-ken(JP)
Inventor: Inoue, Hiromichi
10-2, Otsutomo-cho Kanazawa-ku
Yokohama-shi Kanagawa-ken(JP)
Inventor: Miyazawa, Kazutoshi
12-14, Mutsuura 2-chome Kanazawa-ku
Yokohama-shi Kanagawa-ken(JP)

(74) Representative: Lamb, John Baxter et al
MARKS & CLERK 57/60 Lincoln's Inn Fields
London WC2A 3LS(GB)

(54) **Optically active 2-phenyl-5-alkanoyloxypyrimidines.**

(57) Optically active compounds useful as components of ferroelectric liquid crystal compositions are of the formula

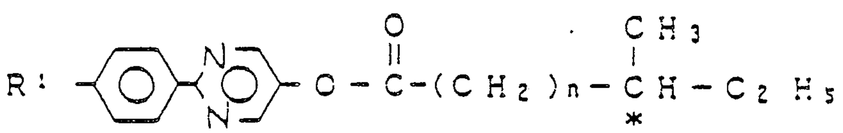

(I)

where $R^1$ is a $C_1$-$C_{20}$ alkyl or $C_1$-$C_{20}$ alkoxy group; is an integer of from 1 to 15; and * indicates an asymmetric carbon atom.

EP 0 325 043 A1

## Optically Active-2-phenyl-5-alkanoyloxypyrimidines

This invention relates to a novel liquid crystal compound having an optically active group and a liquid crystal composition containing the same. More particularly, it relates to an optically active compound useful as a component of ferroelectric liquid crystal compositions and a ferroelectric liquid crystal composition containing the same.

At present, TN (Twisted Nematic) display mode has been most broadly employed. This TN liquid crystal display has a number of advantages such as low driving voltage, small power consumption, etc. However, it is inferior in the aspect of the response rate to emissive display elements such as those of cathodic tube display, electroluminescence display, plasma display, etc. A novel TN mode display element having the twist angle enlarged up to 180° - 270° has also been developed, but it is also still inferior in the response rate. As described above, various efforts for improvement have been made, but no display element having a high response rate has yet been realized. However, a novel display mode the research of which has recently been extensively made and which utilizes ferroelectric liquid crystals has a possibility of notably improving the response rate (Clark et al, Applied Phys. lett., 36, 899 (1980,). This display mode refers to a method utilizing chiral smectic phases such as chiral smectic C phase (hereinafter abbreviated to SC* phase). It has been known that phases exhibiting ferroelectricity are not limited only to SC* phase, but also chiral smectic F, G, H, I phases and the like exhibit ferroelectricity. When these ferroelectric liquid crystals are used for display elements, liquid crystal materials exhibiting ferroelectric liquid crystal phase within a broad temperature range including room temperature have been desired. At present, however, no single compound satisfying such a requirement has yet been known; hence liquid crystal compositions obtained by combining some compounds together and satisfying required characteristics as much as possible have been used.

The object of the present invention is to provide a liquid crystal compound exhibiting ferroelectricity within a broad temperature range including room temperature and useful as a component constituting liquid crystal compositions suitable to the above-mentioned display mode.

The present invention resides in;
an optically active-2-phenyl-5-alkanoyloxypyrimidine expressed by the formula

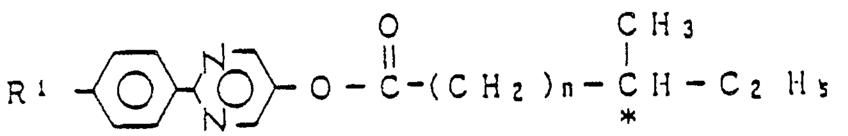 (I)

wherein $R^1$ represents an alkyl group or an alkoxy group each of 1 to 20 carbon atoms, n represents an integer of 1 to 15 and C having a symbol * attached thereonto indicates an asymmetric carbon atom, and also to a liquid crystal composition comprising at least one member of the compound of the formula (I)

In the formula (I), $R^1$ = an alkoxy group of 6 - 15 carbon atoms and n = 2 to 6 are particularly preferred. Concrete examples of compounds having such $R^1$ and n are shown below and phase transition points of representative compounds of the formula (I) are shown in Table 1.

2-(4-Hexylphenyl)-5-(4-methylhexanoyloxy)pyrimidine,
2-(4-Hexylphenyl)-5-(5-methylheptanoyloxy)pyrimidine,
2-(4-Hexylphenyl)-5-(6-methyloctanoyloxy)pyrimidine,
2-(4-Hexylphenyl)-5-(7-methylnonancyloxy)pyrimidine,
2-(4-Hexylphenyl)-5-(8-methyldecanoyloxy)pyrimidine,
2-(4-Heptylphenyl)-5-(4-methylhexanoyloxy)pyrimidine,
2-(4-Heptylphenyl)-5-(5-methylheptanoyloxy)pyrimidine (Compound No. 15),
2-(4-Heptylphenyl)-5-(6-methyloctanoyloxy)pyrimidine,
2-(4-Heptylphenyl)-5-(7-methylnonanoyloxy)pyrimidine,
2-(4-Heptylphenyl)-5-(8-methyldecanoyloxy)pyrimidine,
5-(4-Methylhexanoyloxy)-2-(4-octylphenyl)pyrimidine,
5-(5-Methylheptanoyloxy)-2-(4-octylphenyl)pyrimidine,
5-(6-Methyloctanoyloxy)-2-(4-octylphenyl)pyrimidine (Compound No. 16),
5-(7-Methylnonanoyloxy)-2-(4-octylphenyl)pyrimidine,
5-(8-Methyldecanoyloxy)-2-(4-octylphenyl)pyrimidine,
5-(4-Methylhexanoyloxy)-2-(4-nonylphenyl)pyrimidine,

5-(5-Methylheptanoyloxy)-2-(4-nonylphenyl)pyrimidine,
5-(6-Methyloctanoyloxy)-2-(4-nonylphenyl)pyrimidine,
5-(7-Methylnonanoyloxy)-2-(4-nonylphenyl)pyrimidine,
5-(8-Methyldecanoyloxy)-2-(4-nonylphenyl)pyrimidine,
2-(4-Decylphenyl)-5-(4-methylhexanoyloxy)pyrimidine,
2-(4-Decylphenyl)-5-(5-methylheptanoyloxy)pyrimidine,
2-(4-Decylphenyl)-5-(6-methyloctanoyloxy)pyrimidine,
2-(4-Decylphenyl)-5-(7-methylnonanoyloxy)pyrimidine,
2-(4-Decylphenyl)-5-(8-methyldecanoyloxy)pyrimidine, (Compound No. 17),
5-(4-Methylhexanoyloxy)-2-(4-undecylphenyl)pyrimidine,
5-(5-Methylheptanoyloxy)-2-(4-undecylphenyl)pyrimidine,
5-(6-Methyloctanoyloxy)-2-(4-undecylphenyl)pyrimidine,
5-(7-Methylnonanoyloxy)-2-(4-undecylphenyl)pyrimidine,
5-(8-Methyldecanoyloxy)-2-(4-undecylphenyl)pyrimidine,
2-(4-Docecylphenyl)-5-(4-methylhexanoyloxy)pyrimidine,
2-(4-Dodecylphenyl)-5-(5-methylheptanoyloxy)pyrimidine,
2-(4-Dodecylphenyl)-5-(6-methyloctanoyloxy)pyrimidine,
2-(4-Dodecylphenyl)-5-(7-methylnonanoyloxy)pyimidine,
2-(4-Docecylphenyl)-5-(8-methyldecanoyloxy)pyrimidine,
2-(4-Hexyloxyphenyl)-5-(4-methylhexanoyloxy)pyrimidine,
2-(4-Hexyloxyphenyl)-5-(5-methylheptanoyloxy)pyrimidine,
2-(4-Hexyloxyphenyl)-5-(6-methyloctanoyloxy)pyrimidine (Compound No. 2),
2-(4-Hexyloxyphenyl)-5-(7-methylnonanoyloxy)pyrimidine,
2-(4-Hexyloxyphenyl)-5-(8-methyldecanoyloxy)pyrimidine (Compound No. 3),
2-(4-Heptyloxyphenyl)-5-(4-methylhexanoyloxy)pyrimidine,
2-(4-Heptyloxyphenyl)-5-(5-methylheptanoyloxy)pyrimidine,
2-(4-Heptyloxyphenyl)-5-(6-methyloctanoyloxy)pyrimidine,
2-(4-Heptyloxyphenyl)-5-(7-methylnonanoyloxy)pyrimidine,
2-(4-Heptyloxyphenyl)-5-(8-methyldecanoyloxy)pyrimidine,
5-(4-Methylhexanoyloxy)-2-(4-octyloxyphenyl)pyrimidine (Compound No. 5),
5-(5-Methylheptanoyloxy)-2-(4-octyloxyphenyl)pyrimidine,
5-(6-Methyloctanoyloxy)-2-(4-octyloxyphenyl)pyrimidine,
5-(7-Methylnonanoyloxy)-2-(4-octyloxyphenyl)pyrimidine,
5-(8-Methyldecanoyloxy)-2-(4-octyloxyphenyl)pyrimidine (Compound No. 6),
5-(4-Methylhexanoyloxy)-2-(4-nonyloxyphenyl)pyrimidine,
5-(5-Methylheptanoyloxy)-2-(4-nonyloxyphenyl)pyrimidine,
5-(6-Methyloctanoyloxy)-2-(4-nonyloxyphenyl)pyrimidine,
5-(7-Methylnonanoyloxy)-2-(4-nonyloxyphenyl)pyrimidine (Compound No. 8),
5-(8-Methyldecanoyloxy)-2-(4-nonyloxyphenyl)pyrimidine,
2-(4-Decyloxyphenyl)-5-(4-methylhexanonyloxy)pyrimidine (Compound No. 9),
2-(4-Decyloxyphenyl)-5-(5-methylheptanoyloxy)pyrimidine,
2-(4-Decyloxyphenyl)-5-(6-methyloctanoyloxy)pyrimidine (Compound No. 10),
2-(4-Decyloxyphenyl)-5-(7-methylnonanoyloxy)pyrimidine,
2-(4-Decyloxyphenyl)-5-(8-methyldecanoyloxy)pyrimidine,
5-(4-Methylhexanoyloxy)-2-(4-undecyloxyphenyl)pyrimidine,
5-(5-Methylheptanoyloxy)-2-(4-undecyloxyphenyl)pyrimidine (Compound No. 11),
5-(6-Methyloctanoyloxy)-2-(4-undecyloxyphenyl)pyrimidine,
5-(7-Methylnonanoyloxy)-2-(4-undecyloxyphenyl)pyrimidine,
5-(8-Methyldecanoyloxy)-2-(4-undecyloxyphenyl)pyrimidine (Compound No. 12),
2-(4-Dodecyloxyphenyl)-5-(4-methylhexanoyloxy)pyrimidine (Compound No. 13),
2-(4-Dodecyloxyphenyl)-5-(5-methylheptanoyloxy)pyrimidine,
2-(4-Dodecyloxyphenyl)-5-(6-methyloctanoyloxy)pyrimidine,
2-(4-Dodecyloxyphenyl)-5-(7-methylnonanoyloxy)pyrimidine (Compound No. 14),
2-(4-Dodecyloxyphenyl)-5-(8-methyldecanoyloxy)pyrimidine

Table 1

| Compound No. | In formula (I) | | Absolute configuration | Phase transition points (°C) | | | | Note |
|---|---|---|---|---|---|---|---|---|
| | $R^1$ | n | | C | Sc* | Ch | I | |
| 1 | $C_5H_{11}$-O- | 3 | S | • 85.6 | - | - | • | |
| 2 | $C_6H_{13}$-O- | 4 | S | • 73.8 | - | - | • | |
| 3 | $C_6H_{13}$-O- | 6 | S | • 77.7 | • 81.2 | - | • | |
| 4 | $C_7H_{15}$-O- | 1 | S | • 45.0 | - | • 55.0 | • | |
| 5 | $C_8H_{17}$-O- | 2 | S | • 60.1 | (• 50.0 | • 58.5) | • | Ex. 1 |
| 6 | $C_8H_{17}$-O- | 6 | S | • 62.5 | • 84.8 | - | • | |
| 7 | $C_9H_{19}$-O- | 1 | S | • 46.9 | - | • 57.7 | • | |
| 8 | $C_9H_{19}$-O- | 5 | S | • 59.0 | • 78.2 | - | • | |
| 9 | $C_{10}H_{21}$-O- | 2 | S | • 58.7 | (• 48.5) | • 59.1 | • | |
| 10 | $C_{10}H_{21}$-O- | 4 | S | • 57.6 | • 71.4 | • 73.8 | • | |
| 11 | $C_{11}H_{23}$-O- | 3 | S | • 69.3 | - | (• 63.8) | • | |
| 12 | $C_{11}H_{23}$-O- | 6 | S | • 55.4 | • 86.5 | - | • | |
| 13 | $C_{12}H_{25}$-O- | 2 | S | • 56.8 | (• 49.2) | • 60.1 | • | |
| 14 | $C_{12}H_{25}$-O- | 5 | S | • 58.5 | • 78.4 | - | • | |
| 15 | $C_7H_{15}$- | 3 | S | • 81.0 | - | - | • | Ex. 4 |
| 16 | $C_8H_{17}$- | 4 | S | • 76.0 | - | - | • | Ex. 3 |
| 17 | $C_{10}H_{21}$- | 6 | S | • 74.2 | - | - | • | |

In the Table, C, Ch and I represent crystalline phase, cholesteric phase and isotropic liquid phase, respectively.

Among compounds of the formula (I), those exhibiting ferroelecric liquid crystal phases are, of course, usable by themselves for display element materials, and also preferably usable as a component constituting ferroelectric liquid crystal compositions. Further, even those exhibiting no ferroelectric liquid crystal phase by themselves have a chemical structure similar to those of liquid crystals; hence they are usable as a component constituting liquid crystal compositions. Namely, when the compound of the formula (I) is used for display element materials, the compound of the formula (I) is, of course, usable alone or in admixture with a plurality thereof. Further, it is also possible to mix at least one member of the compound of the formula (I) with at least one member of other optically active or non-optically active smectic liquid crystal compounds, compositions or non-liquid crystal compounds and use the resulting mixtures.

The compound of the formula (I) has specific helical pitches of cholesteric phase and chiral smectic phase respectively, in these phases correspondingly to n, and by choosing an appropriate combination of n in a plurality of the compounds, it is possible to obtain the desired helical pitches of cholesteric phase and chiral smectic phase in the resulting composition.

For commercializing ferroelectric liquid crystal display elements, a technique for adjusting their alignment is important. The alignment properties are said to greatly depend on the chiral pitches of cholesteric and chiral smectic phases, and it is important for adjusting the alignment to obtain desired pitches in these pahses. For example, in the case where the absolute configuration of the compound of the formula (I) is in the form of S, the helical sense of cholesteric phase is right-hand and that of chiral smectic phase is right-hand in the case of n being an odd number, whereas the helical sense of cholesteric phase is left-hand and that of chiral smectic phase is left-hand in the case of n being an even number. Further, the helical pitch of cholesteric phase has a tendency that the larger the number of n, the longer the pitch. Taking these facts into consideration, it is possible to constitute a composition having a desired helical sense and pitch in cholesteric phase and chiral smectic phase. Further, even when the compound is

combined with other optically active compounds, it is possible to obtain a composition having a desired helical sense and pitch in cholesteric phase and chiral smectic phase.

The compound of the present invention has an optically active carbon atom; hence when it is added to nematic liquid crystals, it has as capability of inducing a twisted structure. Nematic liquid crystals having a twisted structure i.e. chiral nematic liquid crystals do not form the so-called reverse domain in TN mode display elements; hence the compound of the present invention is usable as an agent for preventing the reverse domain from forming.

The racemates corresponding to the compound of the formula (I) of the present invention can be prepared in the same manner as in the preparation of the compound of the formula (I) by using raw materials for the racemates. The racemates exhibit almost the same phase transition and phase transition points as those of the corresponding optically active substances, but exhibit SC phase in place of SC* phase and nematic phase (hereinafter abbreviated to N phase) in place of Ch phase. These racemates are usable as an agent for adjusting the chiral pitch of these compounds.

The compound of the formula (I) may be prepared through the following route:

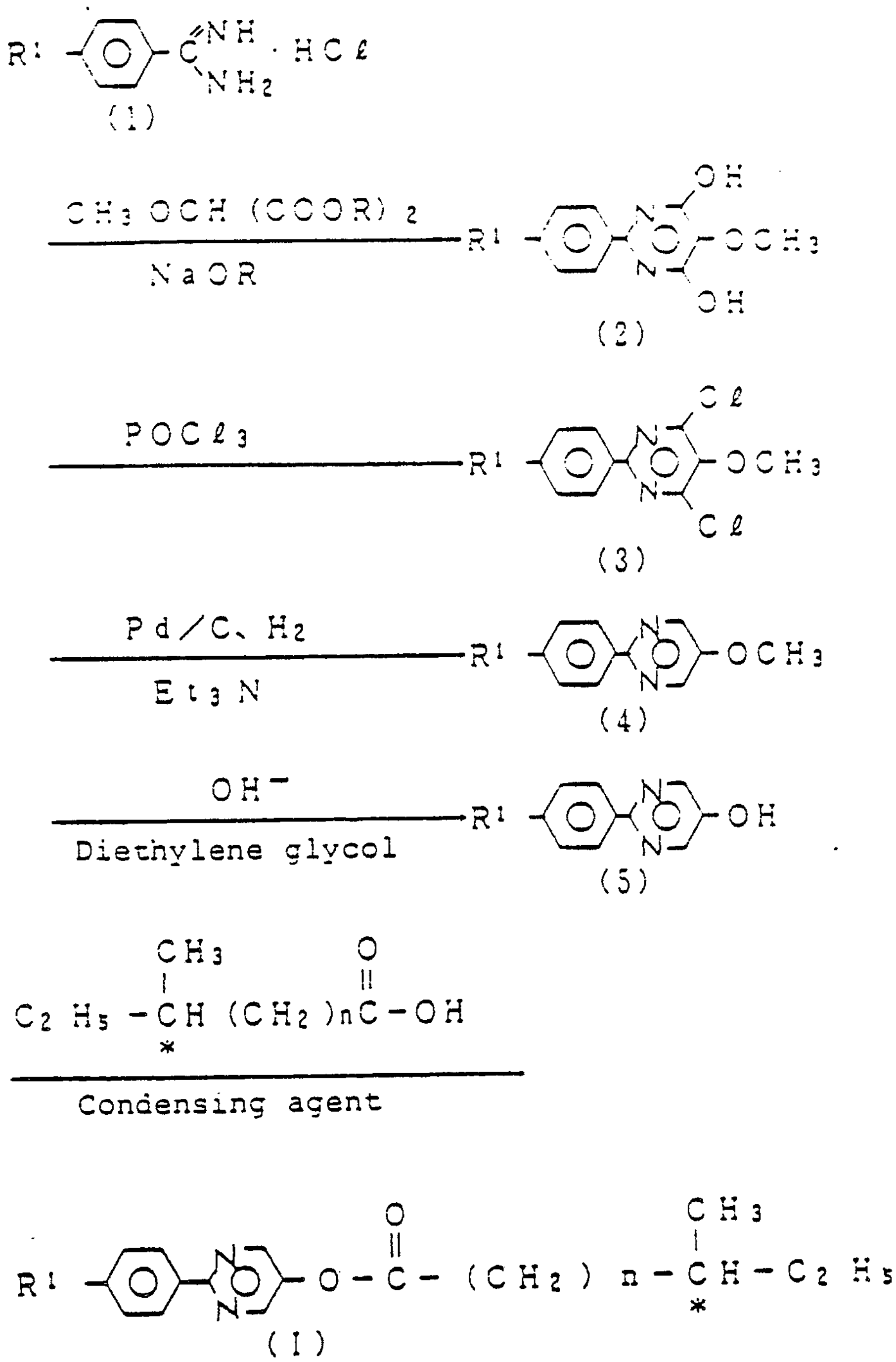


In the above equations, R represents an alkyl group such as methyl group.

Namely, a p-alkylbenzamidine hydrochloride or a p-alkoxybenzamidine hydrochloride (1) is reacted with a methoxymalonic acid diester in the presence of a sodium alcoholate to obtain a diol (2), which is halogen-substituted with a halogenating agent such as phosphorous oxychloride to obtain a compound (3), which is dehalogenated in the presence of a base to obtain a compound (4), which is heat-treated in diethylene glycol to obtain a compound (5), which is esterified with a condensing agent such as dicyclohexylcarbodiimide to obtain the compound of the formula (I).

Further, the compound of the formula (I) can also be prepared through the following route:

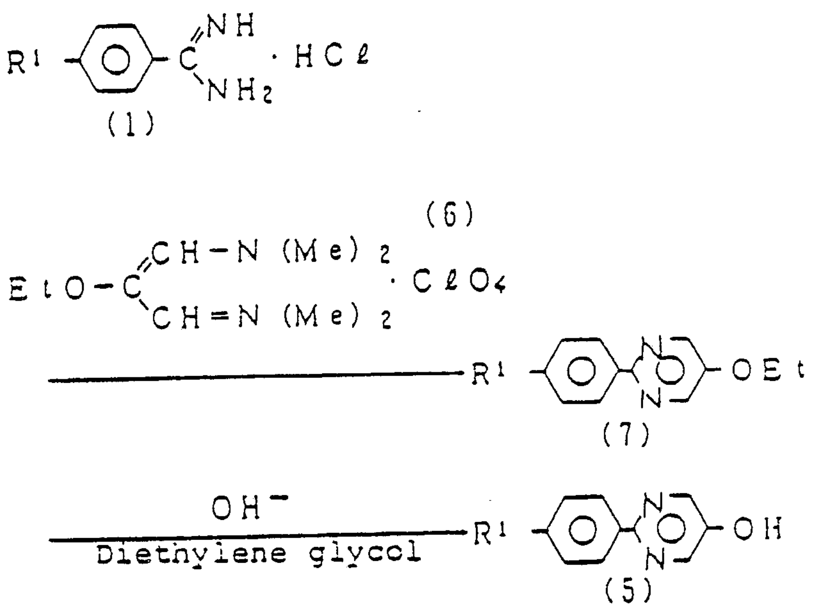


(1)

(7)

(5)

Namely, 1,3,bis(dimethylamino)-2-ethoxytrimethinium perchlorate (6) described in Collection Czechoslov. Chem. Commun., 38 (1973), 1168 is reacted with a p-alkyl benzamidine hydrochloride or a p-alkoxybenzamidine hydrochloride (1) in the presence of a sodium alcoholate to obtain a compound (7), which is treated with an alkali to obtain a compound (5). The subsequent reaction is carried out in the same manner as the above to obtain the compound of the formula (I).

Further, a compound of the formula (I) wherein $R^1$ represents an alkoxy group may also be prepared as follows:

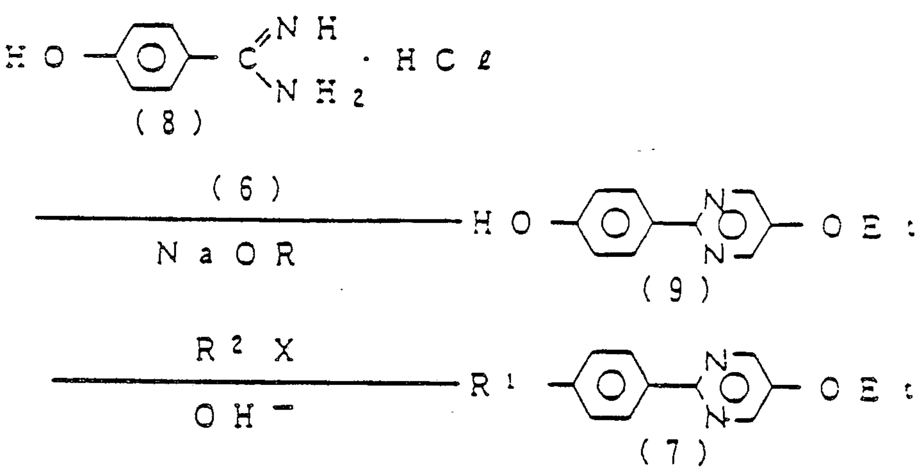


(8)

(9)

(7)

In the above equations, R is as defined before, $R^2$ represents an alkyl group corresponding to $R^1$, and X represents a leaving groupo such as chlorine, bromine, iodine, p-toluenesulfonyloxy group, benzensulfonyloxy group, methanesulfonyloxy group, etc.

Namely, a compound (6) is reacted with a p-hydroxybenzamide hydrochloride (8) in the presence of a sodium alcoholate to obtain a compound (9), which is etherified to obtain a compound (7). The subsequent equations are carried out in the same manner as the above to obtain the compound of (I).

The compound and liquid crystal composition of the present invention will be described in more detail by way of Examples.

Example 1

Preparation of (S)-5-(4-methylhexanoyloxy)-2-(4-octyloxyphenyl)pyrimidine (a compound of the formula (I) wherein $R^1 = C_8H_{17}$-O- and n = 2)

(i) Preparation of 5-ethoxy-2-(4-hydroxyphenyl)-pyrimidine

To a solution of sodium methylate (194.6 g) in methanol (2,000 mℓ) were added p-hydroxybenzamidine hydrochloride (200 g) and 1,3-bis(dimethylamino)-2-ethoxytrimethinium perchlorate (313.5 g) prepared in the same manner as described in Collection Czechoslov. Chem. Commun. 38 (1973), 1168, followed by keeping the mixture under reflux for 6.5 hours, allowing the resulting material to cool down, adding water until the system became uniform, further adding acetic acid until the mixture became acidic and filtering off deposited crystals to obtain 5-ethoxy-2-(4-hydroxyphenyl)-pyrimidine (205 g, m.p.: 201.5 - 202.3° C.

(ii) Preparation of 5-ethoxy-2-(4-octyloxyphenyl)-pyrimidine

To a solution of 5-ethoxy-2-(4-hydroxyphenyl)-pyrimidine (40 g) dissolved in ethanol (340 mℓ) were added KOH (11.2 g) and octyl bromide (35.8 g), followed by keeping the mixture under reflux for 3 hours, distilling off ethanol (about 300 mℓ) , extracting the residue with toluene (500 mℓ), washing the resulting organic layer with 2N-NaOH aqueous solution and then with water until the washing water became neutral, distilling off low boiling substances in the organic layer and recrystallizing the residue from ethanol to obtain 5-ethoxy-2-(4-octyloxyphenyl)pyrimidine (46.9 g). This product exhibited the following phase transition:

$$C \xrightarrow{86.3\,^{\circ}C} N \xrightarrow{93.0\,^{\circ}C} I$$

(iii) Preparation of 5-hydroxy-2-(4-octyloxyphenyl)-pyrimidine

NaOH (58.4 g) was added to a solution of 5-ethoxy-2-(4-octyloxyphenyl)pyrimidine (48 g) dissolved in diethylene glycol (300 mℓ), followed by keeping the mixture at 220° C for 3 hours, allowing the resulting material to cool down, adding acetic acid, filtering off deposited crystals and recrystallizing the residue from ethanol to obtain 5-hydroxy-2-(4-octyloxyphenyl)-pyrimidine (27 g, m.p. 131.2 - 132.5° C).

(iv) Preparation of the captioned compound

5-Hydroxy-2-(4-octyloxyphenyl)pyrimidine (0.9 g), (S)-4-methylhexanoic acid (0.5 g), dicyclohexylcarbodiimide (hereinafter abbreviated to DCC) (1.1 g) and dimethylaminopyridine (hereinafter abbreviated to DMAP) (1.0 g) were dissolved in dichloromethane (30 mℓ), followed by agitating the mixture at room temperature for 4 hours, filtering the reaction mixture, sufficiently washing the resulting organic layer with 2N-NaOH aqueous solution and further with water, distilling off dichloromethane and recrystallizing the residue from ethanol (30 mℓ) to obtain the captioned (S)-5-(4-methylhexanoyloxy)-2-(4-octyloxyphenyl)-pyrimidine (0.4 g). This product exhibited the following phase transition points:

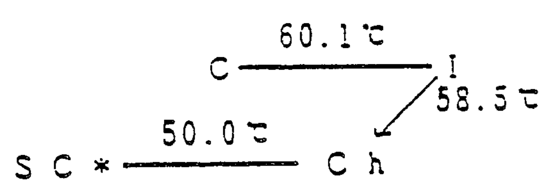


Example 2 (Use example 1)

Using the compounds in the above Table 1, the following composition was prepared:

(Compound 15)

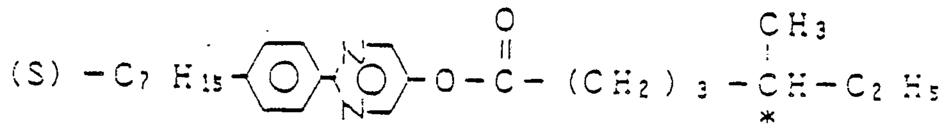

10 wt. %

(Compound 16)

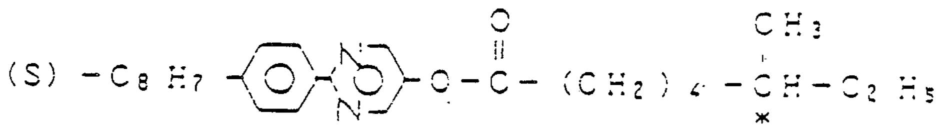

10 wt. %

(Compound 2)

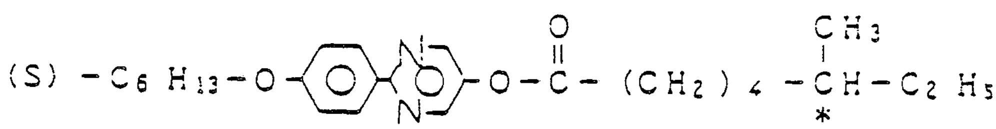

20 wt. %

(Compound 5)

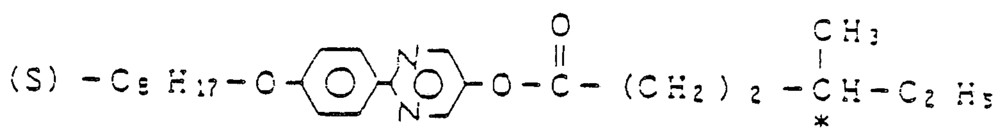

20 wt. %

(Compound 8)

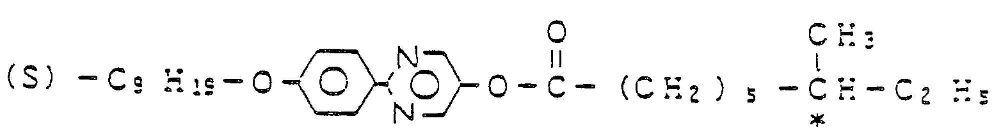

40 wt. %

The above composition exhibited the following phase transition points:

$$C \xrightarrow{40.0^{\circ}C} SC^{*} \xrightarrow{57.8^{\circ}C} Ch \xrightarrow{62.7^{\circ}C} I$$

When this composition was supercooled, SC* phase was observed down to 7.6° C.

As described above, a ferroelectric liquid crystal composition having a broad temperature range including room temperature was obtained.

Example 3 (Use example 2)

A nematic liquid crystal composition consisting of

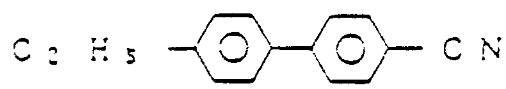

20 wt.%

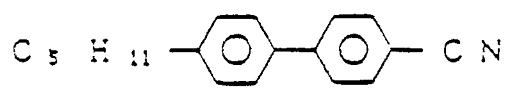

35 wt.%

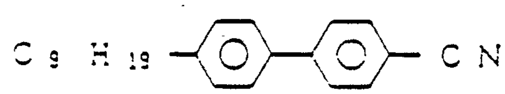

25 wt.% and

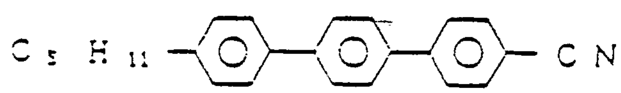

20 wt.%

was filled in a cell provided with transparent electrodes each obtained by coating polyvinyl alcohol (PVA) as an aligning agent and rubbing the resulting surface to subject it to a parallel aligning treatment and having a distance between the electrodes of 10 µm to prepare a TN mode display cell, which was then observed under a polarizing microscope. As a result, a reverse domain was observed to be formed.

To this nematic liquid crystal composition was added a compound of the present invention (Compound No. 16 in Table 1), i.e.

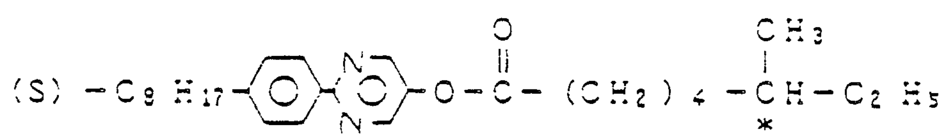

in 1% by weight and a TN mode cell was similarly prepared. When the cell was observed, the reversed domain was dissolved to exhibit a uniform nematic phase.

Example 4 (Use example 3)

To a commercially available nematic liquid crystal composition (ZLI-1132, product made by Merck Company) was added a compound of the present invention (Compound No. 15 in Table 1) i.e.

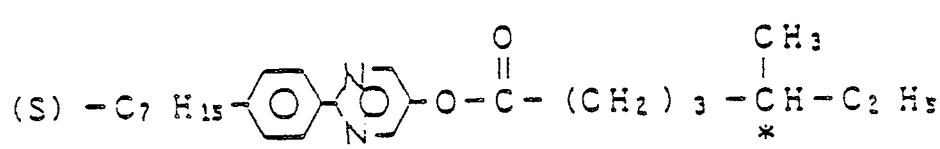

in 1% by weight to prepare a chiral nematic liquid crystal composition, followed by measuring the chiral pitch of the composition according to Cano-Wedge method (see Applied Physics, 43 (2), 126-131 (1974)).

The results were as follows:

| Temperature (°C) | Pitch (μm) |
|---|---|
| 20 | 58.8 |
| 30 | 61.5 |
| 40 | 67.3 |
| 50 | 73.4 |
| 60 | 79.6 |
| 70 | 86.4 |

## Claims

1. Optically active-2-phenyl-5-alkanoyloxypyrimidines of the formula

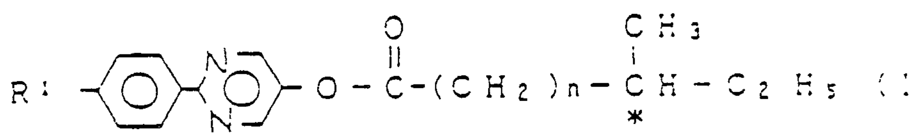

wherein $R^1$ is a $C_1$-$C_{20}$ alkyl or $C_1$-$C_{20}$ alkoxy group; n is an integer of from 1 to 15; and * indicates an asymmetric carbon atom.

2. Compounds according to claim 1 in which $R^1$ is a $C_6$-$C_{15}$ alkoxy group.

3. Compounds according to claim 1 or claim 2 in which n is an integer of from 2 to 6.

4. A liquid crystal composition comprising at least two components, at least one of which is an optically active-2-phenyl-5-alkanoyloxypyrimidine as claimed in claim 1.

5. A liquid crystal composition according to claim 4 which exhibits a chiral smectic phase.

6. A liquid crystal composition according to claim 4, which exhibits a chiral nematic phase.

7. A light switching element comprising a liquid crystal composition as claimed in claim 4.

8. A light switching element comprising a liquid crystal composition as claimed in claim 4 and which exhibits a chiral smectic phase.

European Patent Office

# EUROPEAN SEARCH REPORT

Application Number

EP 88 31 1972

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 191 860 (SEIKO INSTRUMENTS & ELECTRONICS LTD) * claim 1, page 56, lines 5-13 * | 1,4-8 | C 07 D 239/34<br>C 07 D 239/52<br>C 09 K 19/34 |
| A | WO-A-8 606 373 (MERCK PATENT GMBH) * claims * | 1,4-8 | |
| A | EP-A-0 244 129 (AJINOMOTO CO INC) * claims 1,8,9; examples 10-12 * | 1,4-8 | |

TECHNICAL FIELDS SEARCHED (Int. Cl.4)

C 07 D 239/00
C 09 K 19/00

The present search report has been drawn up for all claims

| Place of search | Date of completion of the search | Examiner |
|---|---|---|
| BERLIN | 03-04-1989 | VAN AMSTERDAM L.J.P. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document